# EUROPEAN PATENT SPECIFICATION

(11) **EP 1 317 234 B1**
(45) Date of publication and mention of the grant of the patent: **09.06.2004**
(21) Application number: 01967872.1
(22) Date of filing: 12.09.2001
(51) Int. Cl.: A61F 13/64

(54) **ABSORBENT ARTICLE WITH WAISTBELT ADAPTED FOR DIFFERENT SIZES**
ABSORBIERENDER ARTIKEL MIT TAILLENBÜNDCHEN, WELCHES GEEIGNET IST FÜR UNTERSCHIEDLICHE GRÖSSEN
ARTICLE ABSORBANT DOTE D'UNE CEINTURE S'ADAPTANT A DIFFERENTES TAILLES

(30) Priority: 13.09.2000 SE 0003251
(43) Date of publication of application: 11.06.2003
(73) Proprietor: SCA Hygiene Products AB, 405 03 Göteborg (SE)
(72) Inventor: KARLSSON, Katharina, S-41195 Alingsas (SE)
(74) Representative: Egeröd, Lisbeth
(86) International application number: PCT/SE2001/001947
(87) International publication number: WO 2002/022063

(56) References cited:
- EP-A2- 0 409 307
- EP-A2- 0 972 501
- WO-A1-96/35402
- US-A- 5 989 236

## Description

### Technical field

The present invention refers to an absorbent article such as a diaper and an incontinence guard comprising a liquid permeable topsheet, a liquid impermeable backsheet and an absorbent body enclosed therebetween, said article having a front portion, a rear portion and a crotch portion therebetween, and further is provided with a pair of belt portions attached to the rear portion, alternatively the front portion, of the article and which are intended to be fastened together around the waist of the wearer by means of the first fastening means and where said front portion, alternatively said rear portion, is provided with attachment means intended to be attached to the belt portions, in such a way that the article will assume a pantlike shape, where the belt portions form a part of the waist portions of the pant, wherein the belt portions exhibit an inside intended to be faced towards the user and an outside intended to be faced away from the user.

### Background of the invention

Diapers and incontinence guards for incontinent adults usually have a garment portion holding an absorbent body in place against the user's body and attachment means which hold the garment portion in place also when the user is moving. A common type of attachment means are adhesive tapes or hook and loop fasteners of the touch-and-close type which directly attach the front and rear portions of the absorbent article to each other. It is further known, through e.g., EP-A-0287 388, EP-A-0409 307, EP-A-0 528 282, EP-A-0 605 012 and FR-A-2 586 558, to attach the front and rear portions of the article by means of a belt, at which the possibilities to adjust the fit are improved. The belt further provides a simplified change of diaper or incontinence guard, especially when the patient is standing up.

On a common type of belt diaper the belt portions are first attached around the waist on the patient and then the front portion of the diaper is attached to the outside of the belt using hook and loop fasteners, which hook means are arranged on the front portion of the diaper, wherein the outside portions of the belt function as reception surface or loop material. One problem is that belts of this type have a limited flexibility regarding the possibility to adjust its length. If the belt is applied on a person having a small waist size, the belt portions will reach around the patient all the way to the material on the rear portion of the diaper, where the hook and loop fasteners can not attach. This leads to a poor fit. For a product of one size to fit persons having different waist sizes, it requires a belt allowing an improved adjustment of the waist size. On hospitals and elderly care is this of great importance, since the same diaper size could be used on as many patients as possible.

### Summary of the invention

The object of the present invention is to accomplish a diaper or incontinence guard which can be used on persons having totally different waist sizes. This object is being solved in that one belt portion is provided with a second fastening means on its outside, which is adapted to cooperate with a reception surface on the outside of the rear portion of the article, alternatively to the part of the opposite belt portion connecting to said rear portion. Before the diaper is applied on a user having a small waist size, said one belt portion is folded against the rear portion of the diaper and is attached against the outside of this rear portion. The other belt portion is then alone used as a belt, giving a smaller waist size and has by means of a fastening means the possibility to attach on said back folded belt portion.

### Short description of drawings

The invention will in the following be closer described with reference to an embodiment shown in the accompanying drawings.

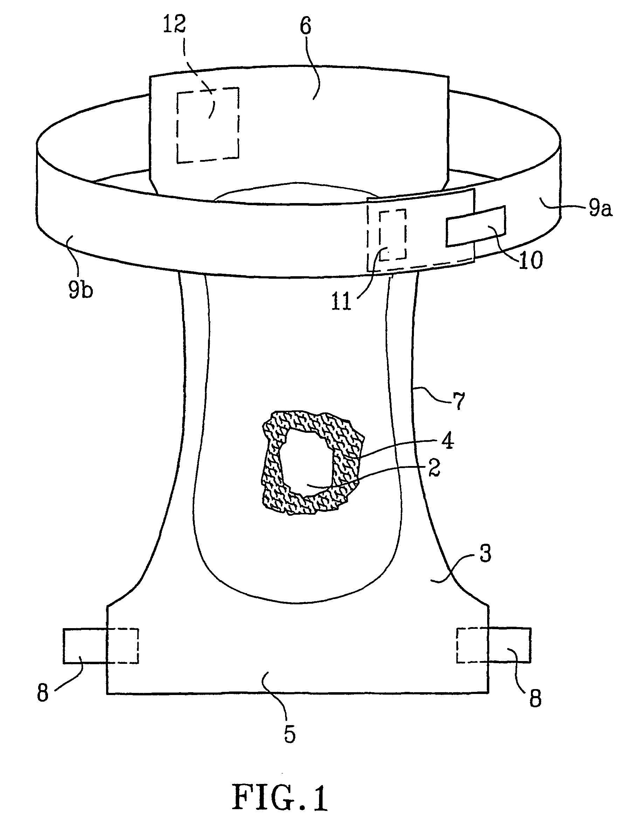

Fig. 1 shows schematically a perspective view of a diaper or incontinence guard according to the invention.

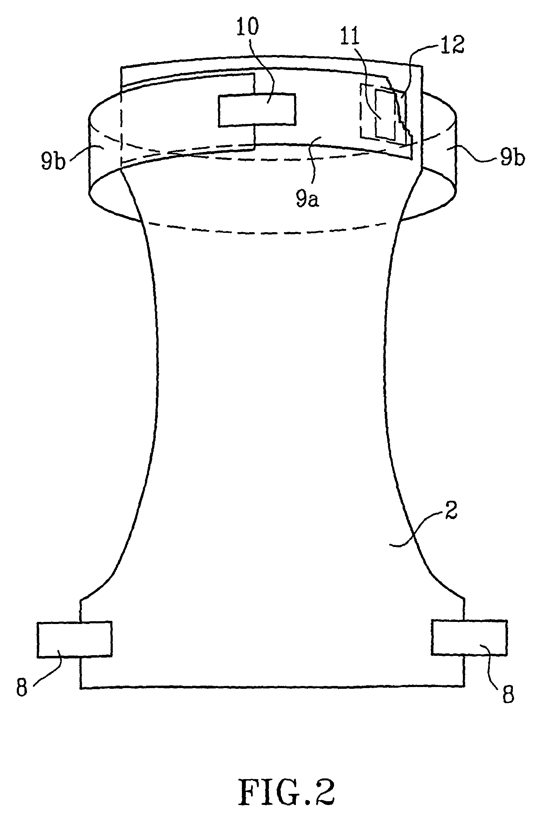

Fig. 2 shows in a rear view the rear portion of a diaper according to Fig. 1.

### Description of an embodiment

The drawings shows an embodiment of a diaper or incontinence guard 1 comprising a liquid impermeable backsheet 2, a liquid permeable topsheet 3 and an absorbent body 4 enclosed therebetween. The liquid permeable topsheet 3 can consist of a nonwoven material, e.g., a spunbond material of continuous filaments, a meltblown material, a bonded carded fibrous web or a perforated plastic film. The liquid impermeable backsheet 2 may consist of a plastic film, a nonwoven material coated with a liquid impervious material or a hydrophobic nonwoven material which resists liquid penetration. In the present invention the backsheet material consists of a plastic film, which can not function as an reception surface for the hook and loop fasteners.

The topsheet 3 and the backsheet material 2 have a somewhat greater extension in the plane than the absorbent body 4 and extend outside the edges thereof. The layers 2 and 3 are connected to each other within the projecting portions thereof, e.g., by gluing or welding by heat or ultrasonic.

The absorbent body 4 can be of any conventional kind Examples of commonly occurring absorbent materials are cellulosic fluff pulp, tissue layers, highly absorbent polymers (so called superabsorbents), absorbent foam materials, absorbent nonwovens or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbent.

The diaper/incontinence guard is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs.

A pair of belt portions 9a, 9b are with one end attached, e. g., glued or ultrasonically welded, to the rear portion 6 of the diaper. The belt portions 9a ,9b are with their opposite ends intended to be fastened together by means of first fastening means 10, comprising one part of a hook-and-loop type fastener and is provided with a surface having hook-material. The outside portions of the belt portions 9 comprise the reception surface for the hook-material. This reception surface, the so-called loop-portion is preferably comprised of a non-woven material. The belt portions 9a, 9b consist of a loop material on both sides. The fastening means 8 of the front portion 5 is intended to be attached against the outsides of the belt portions 9 in order to fasten together the diaper/incontinence guard to the desired pantlike shape. Said fastening means 8 may comprise hook and loop fastener or tape tabs.

According to an alternative embodiment the belt portions are attached to the front portion 5 of the diaper and thus are intended to be fastened together on the back of the wearer. The fastening means 8 are then arranged on the rear portion 6 of the diaper.

The width of the belt portions 9a, 9b should be between 5-20 cm, preferably between 7-15 cm.

The belt portions 9 are preferably a laminate of a carrier material, which forms the outside of the belt, and a soft nonwoven, which forms the inside of the belt, intended to be in direct contact with the skin of the user. A suitable nonwoven material can be a spunbond material of e.g., polypropylene- or polyethylene fibres. Conjugate fibres may also be used. Another suitable nonwoven material can be a carded thermobonded material of e.g., polypropylene-, polyester- or conjugate fibres. The carrier material should be adapted to function as a reception surface for both the attachment means 8 and 10. Also elastic laminates are suitable to use as material in the belt portions.

The belt portions 9a, 9b have loop material on both sides. One belt portion, preferably the left belt portion 9a, is provided with a second fastening means 11, on the side which is intended to be the outside of the belt. This second fastening means 11 preferably consists of a hook material. Before the diaper is applied on a user having a small waist size the belt portion 9a is folded against the outside portion of the rear portion 6 of the diaper. A reception surface 12 is located on the outside of said rear portion. By means of said second fastening means 11 and said reception surface 12 the belt portion 9a is attached against said rear portion 6. The other belt portion 9b may now alone be used as a belt and has the possibility by means of first fastening means 10 to attach against said backfolded belt portion 9a.

This gives a size for said belt product which is smaller than the smallest possible size obtainable using both belt portions. If one wishes to use the product on a bigger person both belt portions 9a,9b are used just as normal, i.e., they are attached around the waist of the user primarily by means of first fastening means 10. One further advantage at normal use of the product i.e, that both fastening means 10, 11 may be used at application which gives a firmer positioning of the belt.

The reception surface 12 preferably consists of a loop material functioning as a reception surface for a hook material. The loop material may for example be a nonwoven material. Alternatively, the part of the belt portion 9b connecting to the rear portion may function as a reception surface 12 for the hook material on the belt portion 9a, whereby no special surface having loop material must be prepared on the back sheet material 2.

The reception surface 12 may also be comprised of a hook material whilst the second fastening means 11 is comprised of a loop material.

In another embodiment the second fastening means 11 on the left belt portions include a tape tab. This tape tab has the capability to attach anywhere on the backsheet material 2. Thereby is it also possible to regulate how much the left belt portion 9a is to be folded back, giving a flexibility in the regulation of the length of the belt.

The location of the fastening means 10, 11 may of course be the other way around, i.e., that the right belt portion 9b is folded back. However, since most people are right handed, the above described embodiments probably are the most preferred.

The diaper may now be adapted to different persons depending on their size with remained fit. It leads to a higher flexibility on for instance hospitals and homes for elderly people, when the same diaper size may be used to an increased number of persons.

The invention is of course not limited to the above described embodiment but can be modified within the scope of the claims.

## Claims

1. Absorbent article such as a diaper and an incontinence guard comprising a liquid permeable topsheet (3), a liquid impermeable backsheet (2) and an absorbent body (4) enclosed therebetween, said article having a front portion (5), a rear portion (6) and a crotch portion (7) therebetween, and further is provided with a pair of belt portions (9a, 9b) attached to either the rear portion (6) or the front portion (5) of the article and which are intended by means of first fastening means (10) to be fastened together around the waist of the wearer and where either said front portion (5) or said rear portion (6) is provided with fastening means (8), intended to be attached to the belt portions (9a, 9b), in such a way that the article will assume a pantlike shape, where the belt portions (9a, 9b) form a part of the waist portions of the pant, whereby the belt portions (9a, 9b) exhibit an inside portion intended to be faced towards the user and an outside portion intended to be faced away from the user,
**characterized in,**
**that** one belt portion (9a) on its outside portion is provided with a second fastening means (11) adapted to cooperate with a reception surface (12) on the outside portic n of either the rear portion (6) or the front portion (5) of the article, and/or on the outside portion of the part of the opposite belt portion (9b) connecting to the rear portion (6) or front portion (5), respectively.

2. Absorbent article according to claim 1,
**characterized in,**
**that** said second fastening means (11) is comprised of a hook material and said reception surface (12) is comprised of a loop material, which together form a hook and loop type fastener.

3. Absorbent article according to claim 1,
**characterized in,**
**that** said second fastening means (11) is comprised of a loop material and said reception surface (12) is comprised of a hook material, which together form a hook and loop type fastener.

4. Absorbent article according to claim 1,
**characterized in,**
**that** said second fastening means (11) is comprised of a tape tab and said reception surface (12) is a part of the backsheet material (2) on the year side of the article.

5. Absorbent article according to claim 1,
**characterized in,**
**that** said second fastening means (11) is comprised of a hook material and said reception surface (12) is comprised of the second belt portion (9b), which together form a hook and loop type fastener.

## Patentansprüche

1. Absorbierender Gegenstand wie eine Windel oder ein Inkontinenzschutz, umfassend eine flüssigkeitsdurchlässige Deckschicht (3), eine flüssigkeitsundurchlässige Tragschicht (2) und einen dazwischen eingeschlossenen, absorbierenden Körper (4), wobei der Gegenstand einen vorderen Abschnitt (5), einen hinteren Abschnitt (6) und einen Schrittabschnitt (7) dazwischen besitzt und ferner mit einem Paar von Gürtelabschnitten (9a, 9b) ausgestattet ist, die an den hinteren Abschnitt (6) oder dem vorderen Abschnitt (5) des Gegenstands angebracht und dazu vorgesehen sind, mittels erster Befestigungsmittel (10) um die Taille des Tragenden befestigt werden, und wobei der vordere Abschnitt (5) oder der hintere Abschnitt (6) mit Befestigungsmitteln (8) ausgestattet ist, die dazu vorgesehen sind, an den Gürtelabschnitten (9a, 9b) derart angebracht zu werden, dass der Gegenstand eine hosenartige Form einnimmt, wobei die Gürtelabschnitte (9a, 9b) einen Teil der Taillenabschnitte der Hose bilden, wobei die Gürtelabschnitte (9a, 9b) einen inneren Abschnitt aufweisen, der dazu vorgesehen ist, dem Benutzer zugewandt zu sein, und einen äußeren Abschnitt aufweisen, der dazu vorgesehen ist, um dem Benutzer abgewandt zu sein,
**dadurch gekennzeichnet,**
**dass** ein Gürtelabschnitt (9a) auf seinem äußeren Abschnitt mit zweiten Befestigungsmitteln (11) ausgestattet ist, die dazu ausgelegt sind, mit einer Aufnahmefläche (12) an dem äußeren Abschnitt des hinteren Abschnitts (6) oder des vorderen Abschnitts (5) des Gegenstands, und/oder an dem äußeren Abschnitt des Teils des gegenüberliegenden Gürtelabschnitts (9b), welcher den hinteren Abschnitt (6) bzw. vorderen Abschnitt (5) verbindet, zusammenzuwirken.

2. Absorbierender Gegenstand nach Anspruch 1,
**dadurch gekennzeichnet,**
**dass** die zweiten Befestigungsmittel (11) ein Hakenmaterial aufweisen, und die Aufnahmefläche (12) weist ein Schlaufenmaterial auf, die gemeinsam einen Klettverschluss bilden.

3. Absorbierender Gegenstand nach Anspruch 1,
**dadurch gekennzeichnet,**
**dass** die zweiten Befestigungsmittel (11) ein Schlaufenmaterial aufweisen, und die Aufnahmefläche (12) weist ein Hakenmaterial auf, die zusammen einen Klettverschluss bilden.

4. Absorbierender Gegenstand nach Anspruch 1,
**dadurch gekennzeichnet,**
**dass** die zweiten Befestigungsmittel (11) eine Bandlasche aufweisen, und die Aufnahmefläche (12) ist ein Teil des Tragschichtmaterials (2) auf der Rückseite des Gegenstands.

5. Absorbierender Gegenstand nach Anspruch 1,
**dadurch gekennzeichnet,**
**dass** die zweiten Befestigungsmittel (11) ein Hakenmaterial aufweisen, und die Aufnahmefläche (12) den zweiten Gürtelabschnitt (9b) aufweist, die zusammen einen Klettverschluss bilden.

## Revendications

1. Article absorbant, tel qu'une couche-culotte et une protection contre l'incontinence, comprenant une feuille supérieure (3) perméable aux liquides, une feuille arrière (2) imperméable aux liquides et un corps absorbant (4) enfermé entre celles-ci, ledit article comportant une partie avant (5), une partie arrière (6) et une partie d'entrejambe (7) située entre celles-ci, et étant en outre muni d'une paire de parties ceinture (9a, 9b) attachées soit à la partie arrière (6) soit à la partie avant (5) de l'article et qui sont destinées à être attachées l'une à l'autre autour de la taille de l'utilisateur, à l'aide de premiers moyens d'attache (10) et dans lequel soit ladite partie avant (5) soit ladite partie arrière (6) est munie de moyens d'attache (8) destinés à être attachés aux parties ceinture (9a, 9b) de telle manière que l'article prenne une forme analogue à celle d'une culotte, les parties ceinture (9a, 9b) constituant une portion des parties taille de la culotte, grâce à quoi les parties ceinture (9a, 9b) présentent une partie intérieure destinée à être orientée en direction de l'utilisateur et une partie extérieure destinée à être
orientée à l'écart de l'utilisateur,
**caractérisé en ce que**
l'une (9a) des parties ceinture est munie sur sa partie extérieure d'un deuxième moyen d'attache (11) adapté pour coopérer avec une surface réceptrice (12) située sur la partie extérieure soit de la partie arrière (6) soit de la partie avant (5) de l'article, et/ou sur la partie extérieure de la portion de la partie ceinture opposée (9b) qui est reliée respectivement soit à la partie arrière (6) soit à la partie avant (5).

2. Article absorbant selon la revendication 1,
**caractérisé en ce que**
ledit deuxième moyen d'attache (11) comprend un matériau doté de crochets et **en ce que** ladite surface réceptrice (12) comprend un matériau doté de boucles, qui forment ensemble une fermeture du type à crochets et à boucles.

3. Article absorbant selon la revendication 1,
**caractérisé en ce que**
ledit deuxième moyen d'attache (11) est constitué d'un matériau doté de boucles et **en ce que** ladite surface réceptrice (12) est constituée d'un matériau doté de crochets, qui forment ensemble une fermeture du type à crochets et à boucles.

4. Article absorbant selon la revendication 1,
**caractérisé en ce que**
ledit deuxième moyen d'attache (11) est constitué d'une patte de ruban et **en ce que** ladite surface réceptrice (12) est une partie du matériau (2) constituant la feuille arrière, située du côté arrière de l'article.

5. Article absorbant selon la revendication 1,
**caractérisé en ce que**
ledit deuxième moyen d'attache (11) est constitué d'un matériau doté de crochets et **en ce que** ladite surface réceptrice (12) est constituée par la deuxième partie ceinture (9b), lesquels forment ensemble une fermeture du type à crochets et à boucles.
